# EUROPÄISCHE PATENTSCHRIFT

(11) **EP 2 252 880 B1**
(45) Veröffentlichungstag und Bekanntmachung des Hinweises auf die Patenterteilung: **28.11.2018**
(21) Anmeldenummer: 09718983.1
(22) Anmeldetag: 20.02.2009
(51) Int. Cl.: G01N 21/89, G01N 33/12

(54) **VORRICHTUNG UND VERFAHREN ZUM KONTAKTLOSEN ERKENNEN VON CHARAKTERISTIKA VON KONTINUIERLICH GEFÖRDERTEN, TRANSLUZENTEN PRODUKTEN**
APPARATUS AND METHOD FOR THE CONTACTLESS DETECTION OF CHARACTERISTICS OF CONTINUOUSLY CONVEYED, TRANSLUCENT PRODUCTS
DISPOSITIF ET PROCÉDÉ POUR DÉTECTER SANS CONTACT DES CARACTÉRISTIQUES DE PRODUITS TRANSLUCIDES TRANSPORTÉS EN CONTINU

(30) Priorität: 08.03.2008 DE 102008013525
(43) Veröffentlichungstag der Anmeldung: 24.11.2010
(73) Patentinhaber: Nordischer Maschinenbau Rud. Baader GmbH + Co. KG, 23560 Lübeck (DE)
(72) Erfinder: SIVERTSEN, Agnar Holten, N-9100 Kvaløysletta (NO); HEIA, Karsten, 9017 Tromsø (NO); NILSEN, Heidi Anita, 9020 Tromsdalen (NO)
(74) Vertreter: Stork Bamberger Patentanwälte PartmbB
(86) Internationale Anmeldenummer: PCT/EP2009/001359
(87) Internationale Veröffentlichungsnummer: WO 2009/112158

(56) Entgegenhaltungen:
- WO-A-2006/075164
- US-A- 4 706 336
- US-A1- 2008 018 892

## Beschreibung

Die Erfindung betrifft eine Vorrichtung zur kontaktlosen Erkennung von Charakteristika von kontinuierlich geförderten, transluzenten Produkten, umfassend zum einen eine Sendeeinheit mit einer Lichtquelle zum Erzeugen hochintensiver Lichtstrahlung, einem Lichtumformelement zur Bildung eines flächigen Lichtfelds aus der Lichtstrahlung und einem Fokussierelement zum Bilden einer quer zur Förderrichtung F der Produkte verlaufenden Lichtlinie aus dem flächigen Lichtfeld, und zum anderen eine Empfangseinheit mit einem Detektionsmittel zum Aufnehmen der vom Produkt transflektierten Lichtstrahlung, wobei zwischen der Sendeeinheit und der Empfangseinheit ein Abschattungselement angeordnet ist.

Des Weiteren betrifft die Erfindung ein Verfahren zum kontaktlosen Erkennen von Charakteristika von kontinuierlich geförderten, transluzenten Produkten, umfassend die Schritte: kontinuierliches Fördern der Produkte durch einen Inspektionsbereich einer Empfangseinheit, Beaufschlagen der Produkte mit Lichtstrahlung mittels einer Sendeeinheit, und Aufnehmen der von den Produkten transflektierten Lichtstrahlung mittels der Empfangseinheit.

Solche Vorrichtungen und Verfahren kommen in unterschiedlichen industriellen Bereichen zum Einsatz, in denen Produkte auf bestimmte Charakteristika untersucht werden. Als Charakteristika kommen unterschiedliche produktspezifische Eigenschaften, aber auch Anomalien oder Fremdkörper und dergleichen in Betracht. In der Fisch verarbeitenden Industrie ist z.B. die Erkennung von Parasiten innerhalb von Fischfilets von besonderer Bedeutung. Das bedeutet, dass jedes Fischfilet auf Parasiten, bei denen es sich üblicherweise um im Verhältnis zum Fischfilet sehr kleine Objekte handelt, untersucht wird. Dabei werden die Fischfilets kontinuierlich mit einer sehr hohen Geschwindigkeit von z.B. 40cm/s und mehr auf einem Transportelement durch einen Inspektionsbereich eines Detektionsmittels gefördert.

Es ist bekannt, die Produkte, im Beispielsfall also die Fischfilets, mit Lichtstrahlung zu beaufschlagen. Die innerhalb des transluzenten Produktes gestreute und/oder reflektierte Lichtstrahlung wird über das Detektionsmittel erfasst und ausgewertet. Anders ausgedrückt dringt die Lichtstrahlung in die Produkte ein, wobei die Lichtstrahlung innerhalb des Produktes z.B. an Fremdkörpern ungerichtet gestreut oder gerichtet reflektiert wird. Zusätzlich können auch weitere Prozesse, wie z.B. Absorption und Fluoreszenz die spektrale Charakteristik des Lichts ändern. Diese als Ergebnis der Transflektion (transflectance/interactance) erhaltene Lichtstrahlung (gestreut und/oder gerichtet) wird im Folgenden auch als transflektiertes Licht bezeichnet. Das transflektierte Licht wird dann vom Detektionsmittel erfasst. Bekannte Vorrichtungen sind derart ausgebildet, dass sie eine Sendeeinheit mit einer Lichtquelle aufweisen, wobei die Lichtquelle hochintensive Lichtstrahlung in ein Lichtumformelement strahlt. Innerhalb des Lichtumformelementes, in dem z.B. Glasfaserbündel angeordnet sein können, wird die Lichtstrahlung geformt und von der Lichteintrittsöffnung an die Lichtaustrittsöffnung geleitet. Das Lichtumformelement weist im Austrittsbereich für die Lichtstrahlung eine flächige Öffnung auf. Aus physikalischen Gründen streut die Lichtstrahlung beim Austritt aus dem Lichtumformelement. Dieses Streulicht trifft dann auf das unterhalb des Lichtumformelementes angeordnete Fokussierelement, das das flächige Lichtfeld zu einer Lichtlinie fokussiert, die quer zur Förderrichtung F der Produkte verläuft. Bei den bekannten Vorrichtungen ist die Sendeeinheit in Förderrichtung F der Produkte entweder vor der Empfangseinheit oder hinter der Empfangseinheit angeordnet.

Das Problem bei den existierenden Vorrichtungen besteht darin, dass die einzige Sendeeinheit und damit die einzige Lichtquelle einerseits eine zu geringe Lichtintensität aufweist, um das Produkt bei hohen Fördergeschwindigkeiten ausreichend zu Be-/Durchleuchten, und andererseits nur einen begrenzten Bereich des Fischfilets ausleuchtet. Das führt insbesondere dazu, dass Teilbereiche des Fischfilets, nämlich entweder im Voraus laufenden Bereich oder im nach laufenden Bereich, nicht beleuchtet sind, wodurch eine vollständige Inspektion unmöglich ist.

Es ist daher Aufgabe der vorliegenden Erfindung, eine Vorrichtung zu schaffen, die die Detektion von Charakteristika von transluzenten Produkten bei hoher Fördergeschwindigkeit der Produkte zuverlässig gewährleistet. Des Weiteren ist es Aufgabe der Erfindung, ein entsprechendes Verfahren vorzuschlagen.

Diese Aufgabe wird durch eine Vorrichtung mit den eingangs genannten Merkmalen dadurch gelöst, dass mindestens zwei Sendeeinheiten mit entsprechendem Aufbau vorgesehen sind, derart, dass mindestens zwei unabhängige Lichtquellen zum Beleuchten des Produktes vorgesehen sind, wobei eine Sendeeinheit in Förderrichtung F der Produkte vor der Empfangseinheit und die andere Sendeeinheit in Förderrichtung F hinter der Empfangseinheit angeordnet ist, wobei beide Sendeeinheiten gegenüber der Empfangseinheit mittels eines Abschattungselementes abgeschirmt sind, wobei die Empfangseinheit sandwichartig zwischen den vertikal zur Förderebene der Produkte gerichteten Beschattungselementen angeordnet ist. Damit wird auf überraschend einfache und zuverlässige Weise die Ausleuchtung des gesamten Produktes sichergestellt. Neben der Erhöhung der Lichtintensität durch mehrere Lichtquellen stellt die erfindungsgemäße Anordnung der Lichtquellen auch sicher, dass das Produkt flächenmäßig ausreichend ausgeleuchtet wird. Sowohl beim Einlaufen der Produkte in den Inspektionsbereich als auch beim Auslaufen aus dem Inspektionsbereich ist eine Beaufschlagung des zu inspizierenden Produktes mit Licht sichergestellt. Auch sorgt die erhöhte Lichtintensität für eine verbesserte Durchleuchtung des Produktes. Anders ausgedrückt dringt das Licht tief in die Produkte ein. Die Abschattungselemente stellen sicher, dass Lichtstrahlung, die von den Linsen in Richtung des Inspektionsbereiches gestreut wird, zuverlässig abgeschirmt wird, so dass eine Beeinträchtigung der Aufnahme des vom bzw. genauer aus dem Produkt und/oder vom Transportelement und/oder von der Vorrichtung selbst reflektierten Lichtes ausgeschlossen ist.

Vorteilhafterweise sind jeder Sendeeinheit mindestens zwei Abschattungselemente zugeordnet, derart, dass die Sendeeinheiten jeweils zu beiden Seiten mittels eines Abschattungselementes abgeschirmt sind. Durch diese erfindungsgemäße Ausbildung wird verhindert, dass Streulicht auf der der Empfangseinheit abgewandten Seite den Inspektionsbereich beeinträchtigt.

Eine besonders bevorzugte Ausführungsform ist dadurch gekennzeichnet, dass das Fokussierelement eine zylinderförmige Linse ist. Damit wird auf einfache Weise eine linienförmige Lichtstrahlung erzeugt, so dass die Produkte beim Durchlaufen des Inspektionsbereiches über ihre gesamte Breite quer zur Förderrichtung F mit Licht beaufschlagt werden.

Eine bevorzugte Ausgestaltung ist dadurch gekennzeichnet, dass die Linsen der beiden Sendeeinheiten parallel zueinander ausgerichtet sind. Damit wird eine optimale Ausleuchtung des Inspektionsbereiches erreicht, und zwar für das gesamte Produkt. Anders ausgedrückt wird ein beleuchtungsfreier Abschnitt des kontinuierlich geförderten Produkts mit der beschriebenen Ausgestaltung ausgeschlossen.

Besonders vorteilhaft ist eine Vorrichtung, die sich dadurch auszeichnet, dass der Abstand zwischen den beiden durch die Lichtquellen bzw. die Linsen erzeugten Lichtlinien etwa 40mm beträgt. Damit wird der zuvor angesprochene Vorteil noch weiter unterstützt.

Die Aufgabe wird auch durch ein Verfahren mit den eingangs genannten Schritten dadurch gelöst, dass die Produkte sowohl beim Einlaufen in den Inspektionsbereich eines Detektionsmittels der Empfangseinheit als auch beim Auslaufen aus dem Inspektionsbereich des Detektionsmittels durch separate Lichtquellen mit einer hochintensiven Lichtstrahlung beaufschlagt werden. Die sich daraus ergebenden Vorteile wurden im Zusammenhang mit der Vorrichtung beschrieben, so dass zur Vermeidung von Wiederholungen auf die entsprechenden Passagen verwiesen wird.

Weitere zweckmäßige und/oder vorteilhafte Merkmale und Weiterbildungen ergeben sich aus den Unteransprüchen und der Beschreibung. Eine besonders bevorzugte Ausgestaltung wird anhand der beigefügten Zeichnung näher erläutert. In der Zeichnung zeigt:
- Fig. 1: eine schematische Darstellung einer ersten Ausführungsform der erfindungsgemäßen Vorrichtung in Vorderansicht,
- Fig. 2: eine schematische Darstellung einer weiteren Ausführungsform der erfindungsgemäßen Vorrichtung in Vorderansicht, und

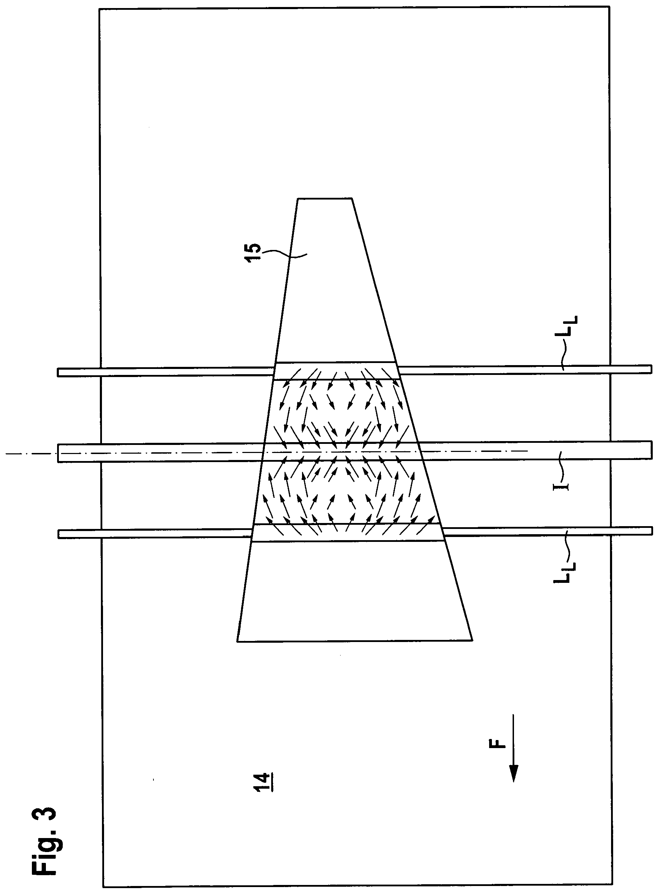

- Fig. 3: eine Draufsicht auf ein zu inspizierendes Produkt mit angedeuteten Lichtlinien und angedeutetem Inspektionsbereich.

Die beschriebenen Vorrichtungen dienen zum Erkennen von Parasiten in Fischfilets. Die Vorrichtung ist jedoch gleichermaßen zur Erkennung von Charakteristika anderer transluzenter Produkte geeignet.

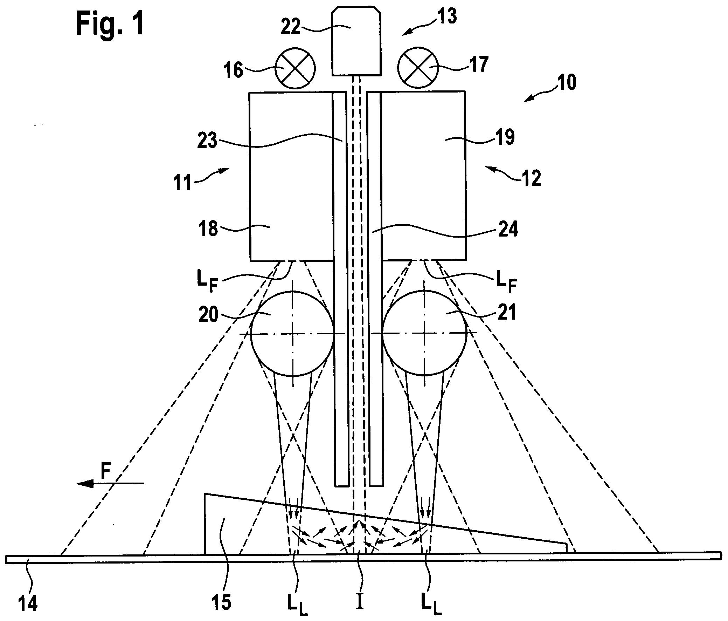

Die in Figur 1 gezeigte Vorrichtung 10 umfasst eine erste Sendeeinheit 11, eine zweite Sendeeinheit 12 sowie eine Empfangseinheit 13. Die Vorrichtung 10 ist üblicherweise oberhalb eines Transportelementes 14 angeordnet. Eine Anordnung unterhalb des Transportelementes 14 ist jedoch ebenfalls möglich. Auf dem Transportelement 14 werden die Produkte 15 kontinuierlich und mit hoher Geschwindigkeit in Förderrichtung F gefördert, wobei die Förderrichtung F auch umgekehrt gerichtet sein kann.

Jede Sendeeinheit 11, 12 weist eine Lichtquelle 16 bzw. 17 zum Erzeugen einer hochintensiven Lichtstrahlung, ein Lichtumformelement 18 bzw. 19 zur Bildung eines flächigen Lichtfeldes aus der von den Lichtquellen 16, 17 ausgehenden Lichtstrahlung sowie ein Fokussierelement 20 bzw. 21 zum Bilden einer quer zur Förderrichtung F der Produkte 14 verlaufenden Lichtlinie L_{L} aus dem flächigen Lichtfeld auf. Die Empfangseinheit 13 weist ein Detektionsmittel 22 auf, das z.B. eine Kamera oder dergleichen sein kann. Jede Lichtquelle 16, 17 kann innerhalb oder außerhalb des Lichtumformelementes 18, 19 angeordnet und zum Beispiel aus mehreren, vorzugsweise drei Halogenlampen (z.B. mit einer Leistung von jeweils 150W und einer Farbtemperatur von ca. 3200 K) mit zugeordneten Reflektoren gebildet sein.

In der in Figur 1 gezeigten Ausführungsform ist zwischen den Sendeeinheiten 11, 12 und der Empfangseinheit 13 jeweils ein Abschattungselement 23 bzw. 24 vorgesehen. Die Abschattungselemente 23, 24 sind lichtundurchlässig ausgebildet und erstrecken sich quer zur Förderrichtung F über die gesamte Breite der Vorrichtung 10 und vertikal zur Förderebene vom Detektionsmittel 22 bis kurz über die zu inspizierenden Produkte 15. Die Länge der Abschattungselement 23, 24 kann insbesondere in vertikaler Erstreckung variieren.

Die separaten Sendeeinheiten 11, 12 sind zu beiden Seiten der Empfangseinheit 13 angeordnet. In Förderrichtung F der Produkte 15 ist eine der Sendeeinheiten 11, 12 vor der Empfangseinheit 13 und die andere Sendeeinheit 12, 11 hinter der Empfangseinheit 13 angeordnet. Damit ist die Empfangseinheit 13 jeweils getrennt durch die Abschattungselemente 23, 24 sandwichartig zwischen den Sendeeinheiten 11, 12 angeordnet. Weitere Anordnungen der Sendeeinheiten 11, 12 in Bezug auf die Empfangseinheit 13, beispielsweise versetzt zur Empfangseinheit 13, sind ebenfalls möglich. Wie bereits erwähnt, sind beide Sendeeinheiten 11, 12 bevorzugt oberhalb des Transportelementes 14 angeordnet. Möglich ist aber auch eine Anordnung beider Sendeeinheiten 11, 12 unterhalb des dann lichtdurchlässigen Transportelementes 14 bzw. eine variable Anordnung mit einer Sendeeinheit 11 oder 12 oberhalb und einer Sendeeinheit 12 oder 11 unterhalb des Transportelementes 14.

Die Fokussierelemente 20, 21 sind bevorzugt als zylinderförmige Linse ausgebildet. Die Ausgestaltung und Dimensionierung der Linsen kann variieren. Bevorzugt ist eine Linse mit einem Durchmesser von etwa 25mm und einer Länge von etwa 200mm. Die Linsen bestehen bevorzugt aus Poly(meth)acrylaten. Andere geeignete Werkstoffe sind jedoch ebenfalls möglich. Die Linsen sind jeweils lösbar an dem Lichtumformelement 18, 19 befestigt, beispielsweise durch Klammern oder dergleichen. Die Linsen verlaufen vorzugsweise parallel zueinander. Anders ausgedrückt sind die Mittelachsen beider Linsen quer zur Förderrichtung F der Produkte 15 ausgerichtet. Der Abstand der Linsen zueinander kann in Abhängigkeit unterschiedlicher Faktoren (z.B. Produktgröße) variieren. Ein besonders bevorzugter Abstand ist derart gewählt, dass die durch die Lichtquellen 16, 17 bzw. die Lichtumformelemente 18, 19 und die Fokussierelemente 20, 21 erzeugten Lichtlinien L_{L} einen Abstand von etwa 40mm aufweisen.

Das Detektionsmittel 22 umfasst eine Kamera. Optional kann auch eine Sensoreinheit vorgesehen sein. Selbstverständlich sind auch alle anderen bekannten Elemente zum Empfangen von Signalen und insbesondere zur Erkennung und Aufnahme von transflektiertem Licht einsetzbar. Bevorzugt kann das Detektionsmittel 22 auch ein Spektrometer mit Ortsauflösung sein. Bevorzugt ist ein Spektrometer mit 128 Spektralbändern in einem Bereich von 400 bis 1000nm und einer räumlichen Auflösung (Ortsauflösung) von ca. 0,5mm² (0,5mm quer zur Förderrichtung F und 1mm in Förderrichtung F) vorgesehen. Die bevorzugte Lesegeschwindigkeit beträgt 400Hz. Das Spektrometer ist in einem definierten Abstand zum Transportelement 14 angeordnet. Der Abstand kann variieren, beträgt bevorzugt jedoch etwa 1000mm. Dem Detektionsmittel 22 kann eine (nicht dargestellte) Auswerteeinheit zugeordnet sein. Bestandteil der Auswerteeinheit kann des Weiteren eine Computereinheit und/oder eine Steuereinheit sein, mittels der auf die ausgewerteten Ergebnisse reagiert werden kann, beispielsweise zur Ausschleusung unerwünschter oder fehlerhafter Produkte oder dergleichen.

In den beschriebenen Ausführungsformen ist das Lichtumformelement 18, 19 eine quaderförmige Box mit den bevorzugten Abmessungen Breite x Höhe x Länge von 25 x 100 x 200mm. Selbstverständlich sind die Abmessungen nahezu beliebig variabel. Die Box ist aus einem lichtundurchlässigen Material, wie z.B. schwarz lackiertem Aluminium, hergestellt und weist innerhalb des Innenraums mehrere, vorzugsweise drei Glasfaserbündel auf. Die Box verfügt über eine Lichteintrittsöffnung. Im Bereich dieser Lichteintrittsöffnung ist die Lichtquelle 16, 17 angeordnet. Des Weiteren verfügt die Box über eine Lichtaustrittsöffnung, die auf der den Linsen zugewandten Seite ausgebildet ist. Die Lichtaustrittsöffnung ist vorzugsweise rechteckförmig ausgebildet, so dass das von den Glasfaserbündeln von der Lichteintrittsöffnung zur Lichtaustrittsöffnung geleitete Licht in einem flächigen Lichtfeld L_{F} (oder auch Lichtband) aus der Box austritt. Die Größe und Form der Lichtaustrittsöffnung kann selbstverständlich variieren. Neben der bevorzugten Ausgestaltung sind selbstverständlich auch andere Ausbildungen und Ausführungen des Lichtumformelementes 18, 19 möglich.

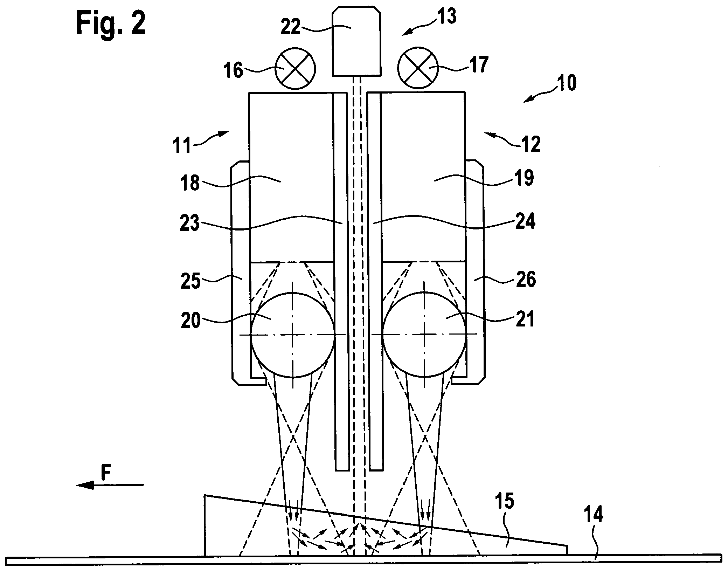

In weiteren Ausführungsformen können z.B. zusätzliche Abschattungselemente 25 bzw. 26 vorgesehen sein. In der Ausführungsform gemäß Figur 2 sind jeder Sendeeinheit 11, 12 mindestens zwei Abschattungselemente 23 und 25 bzw. 24 und 26 zugeordnet. Die Abschattungselemente 23, 24 dienen der Abschirmung der Linsen auf der dem Inspektionsbereich zugewandten Seite. Die Abschattungselemente 25, 26 dienen der Abschirmung der Linsen auf der gegenüber liegenden Seite. Sämtliche Abschattungselemente 23 bis 26 bestehen bevorzugt aus schwarz lackiertem Aluminium. Andere Ausbildungen sind aber ebenfalls möglich. Für den Fall, dass nur die dem Inspektionsbereich I zugewandten Abschattungselemente 23, 24 vorgesehen sind, erstrecken diese sich bis kurz über die zu fördernden Produkte. Die dem Inspektionsbereich I abgewandten Abschattungselemente 25, 26 weisen zusätzlich zu den vertikal verlaufenden Abschnitten noch einen horizontal verlaufenden Abschnitt auf, der die Linsen nach unten hin teilweise abschirmt. Insgesamt dienen die Abschattungselemente 23 bis 26 dazu, dass von den Linsen streuende Licht zu reduzieren.

Das Verfahrensprinzip wird anhand der Figuren im Folgenden erläutert:
Die Produkte 15, also z.B. die Fischfilets, werden in Förderrichtung F auf dem Transportelement 14 mit hoher Geschwindigkeit gefördert. Beim Einlaufen der Produkte 15 in den Inspektionsbereich I wird der voraus laufende Abschnitt des Produktes 15 bereits durch die erste Sendeeinheit 12 mit Licht beaufschlagt, so dass die Empfangseinheit 13 transflektiertes Licht, also Licht, das innerhalb des Produktes streut und/oder gerichtet reflektiert wird, aufnehmen kann. Das kontinuierlich geförderte Produkt 15 wird dann weiter durch den Inspektionsbereich I hindurch zum Auslaufbereich gefördert. Wenn sich der nachlaufende Abschnitt des Produktes 15 gerade noch im Inspektionsbereich I befindet, wird der nachlaufende Bereich noch durch die zweite Sendeeinheit 11 mit Licht beaufschlagt. Dazwischen beleuchten beide Sendeeinheiten 11, 12 parallel das Produkt 15.

Die Anordnung der Vorrichtung 10 stellt demnach sicher, dass die zu inspizierenden Produkte einerseits vollständig beleuchtet werden und andererseits diese Beleuchtung auch mit der notwendigen Intensität erfolgt. Die Produkte 15 können auch in umgekehrter Förderrichtung gefördert werden.

## Patentansprüche

1. Vorrichtung (10) zur kontaktlosen Erkennung von Charakteristika von kontinuierlich geförderten, transluzenten Produkten (15), umfassend zum einen eine Sendeeinheit (11, 12) mit einer Lichtquelle (16, 17) zum Erzeugen hochintensiver Lichtstrahlung, einem Lichtumformelement (18, 19) zur Bildung eines flächigen Lichtfelds aus der Lichtstrahlung und einem Fokussierelement (20, 21) zum Bilden einer quer zur Förderrichtung F der Produkte (15) verlaufenden Lichtlinie aus dem flächigen Lichtfeld, und zum anderen eine Empfangseinheit (13) mit einem Detektionsmittel (22) zum Aufnehmen der vom Produkt (15) transflektierten Lichtstrahlung, wobei zwischen der Sendeeinheit (11, 12) und der Empfangseinheit (13) ein Abschattungselement (23, 24) angeordnet ist, **dadurch gekennzeichnet, dass** mindestens zwei Sendeeinheiten (11, 12) mit entsprechendem Aufbau vorgesehen sind, derart, dass mindestens zwei unabhängige Lichtquellen (16, 17) zum Beleuchten des Produktes (15) vorgesehen sind, wobei eine Sendeeinheit (12) in Förderrichtung F der Produkte (15) vor der Empfangseinheit (13) und die andere Sendeeinheit (11) in Förderrichtung F hinter der Empfangseinheit (13) angeordnet ist, wobei beide Sendeeinheiten (11, 12) gegenüber der Empfangseinheit (13) mittels eines Abschattungselementes (23, 24) abgeschirmt sind, wobei die Empfangseinheit (13) sandwichartig zwischen den vertikal zur Förderebene der Produkte (15) gerichteten Abschattungselementen (23, 24) angeordnet ist.

2. Vorrichtung nach Anspruch 1, **dadurch gekennzeichnet, dass** jeder Sendeeinheit (11, 12) mindestens zwei Abschattungselemente (23, 25; 24, 26) zugeordnet sind, derart, dass die Sendeeinheiten (11, 12) jeweils zu beiden Seiten mittels eines Abschattungselementes (23, 25; 24, 26) abgeschirmt sind.

3. Vorrichtung nach Anspruch 1 oder 2, **dadurch gekennzeichnet, dass** das Fokussierelement (20, 21) eine zylinderförmige Linse ist.

4. Vorrichtung nach Anspruch 3, **dadurch gekennzeichnet, dass** die Linse aus Poly(meth)acrylaten besteht und einen Durchmesser von 25mm und eine Länge von 200mm aufweist.

5. Vorrichtung nach Anspruch 3 oder 4, **dadurch gekennzeichnet, dass** die Linse am Lichtumformelement (18, 19) befestigt ist.

6. Vorrichtung nach einem der Ansprüche 3 bis 5, **dadurch gekennzeichnet, dass** die Linsen der beiden Sendeeinheiten (11, 12) parallel zueinander ausgerichtet sind, wobei die Mittelachsen der Linsen quer zur Förderrichtung F der Produkte (15) ausgerichtet sind.

7. Vorrichtung nach einem der Ansprüche 1 bis 6, **dadurch gekennzeichnet, dass** der Abstand zwischen den beiden durch die Lichtquellen (16, 17) bzw. die Linsen erzeugten Lichtlinien etwa 40mm beträgt.

8. Vorrichtung nach einem der Ansprüche 1 bis 7, **dadurch gekennzeichnet, dass** das Detektionsmittel (22) eine Kamera umfasst, wobei dem Detektionsmittel (22) eine Auswerteeinheit zugeordnet ist.

9. Vorrichtung nach einem der Ansprüche 1 bis 8, **dadurch gekennzeichnet, dass** das Lichtumformelement (18, 19) zur Bildung eines rechteckförmigen Lichtfeldes ausgebildet und eingerichtet ist.

10. Vorrichtung nach einem der Ansprüche 1 bis 9, **dadurch gekennzeichnet, dass** beide Sendeeinheiten (11, 12) oberhalb eines Transportelementes (14) zum Fördern der Produkte (15) angeordnet sind.

11. Verfahren zum kontaktlosen Erkennen von Charakteristika von kontinuierlich geförderten, transluzenten Produkten (15), mit einer Vorrichtung nach einem oder mehreren der Ansprüche 1 bis 10, umfassend die Schritte:
- kontinuierliches Fördern der Produkte (15) durch einen Inspektionsbereich einer Empfangseinheit (13),
- Beaufschlagen der Produkte (15) mit Lichtstrahlung mittels einer Sendeeinheit (11, 12), und
- Aufnehmen der von den Produkten (15) transflektierten Lichtstrahlung mittels der Empfangseinheit (13),
**dadurch gekennzeichnet, dass** die Produkte (15) sowohl beim Einlaufen in den Inspektionsbereich eines Detektionsmittels (22) der Empfangseinheit (13) als auch beim Auslaufen aus dem Inspektionsbereich des Detektionsmittels (22) durch separate Lichtquellen (16, 17) mit einer hochintensiven Lichtstrahlung beaufschlagt werden.

12. Verfahren nach Anspruch 11, **dadurch gekennzeichnet, dass** die von den Lichtquellen (16, 17) ausgehenden Lichtstrahlen zunächst von einem Lichtumformelement (18, 19) zu einem flächigen Lichtfeld aufgeweitet und dann von einem Fokussierelement (20, 21) zu einer quer zur Förderrichtung F der Produkte (15) verlaufenden Lichtlinie fokussiert werden, wobei die Lichtstrahlen zu mindestens zwei Seiten abgeschirmt werden.

13. Verfahren nach Anspruch 11 oder 12, **dadurch gekennzeichnet, dass** die mittels der Empfangseinheit (13) aufgenommene, transflektierte Lichtstrahlung mittels einer Auswerteeinheit verarbeitet wird.

## Claims

1. Apparatus (10) for the contactless detection of characteristics of continuously conveyed, translucent products (15), comprising firstly a transmitting unit (11, 12) having a light source (16, 17) for generating high-intensity light radiation, a light-converting element (18, 19) for forming a planar light field from the light radiation and a focusing element (20, 21) for forming a line of light running transversely to the direction of conveying F of the products (15) from the planar light field, and secondly a receiving unit (13) having a detection means (22) for picking up the light radiation transflected by the product (15), wherein a shading element (23, 24) is arranged between the transmitting unit (11, 12) and the receiving unit (13), **characterised in that** at least two transmitting units (11, 12) with corresponding structure are provided, such that at least two independent light sources (16, 17) for illuminating the product (15) are provided, wherein one transmitting unit (12) is arranged in front of the receiving unit (13) in the direction of conveying F of the products (15) and the other transmitting unit (11) is arranged behind the receiving unit (13) in the direction of conveying F, wherein the two transmitting units (11, 12) are shielded from the receiving unit (13) by means of a shading element (23, 24), wherein the receiving unit (13) is sandwiched between the shading elements (23, 24) which are directed vertically to the plane of conveying of the products (15).

2. Apparatus according to claim 1, **characterised in that** each transmitting unit (11, 12) is assigned at least two shading elements (23, 25; 24, 26), such that the transmitting units (11, 12) are shielded by means of a shading element (23, 25; 24, 26) on either side.

3. Apparatus according to claim 1 or 2, **characterised in that** the focusing element (20, 21) is a cylindrical lens.

4. Apparatus according to claim 3, **characterised in that** the lens is made of poly(meth)acrylates and has a diameter of 25 mm and a length of 200 mm

5. Apparatus according to claim 3 or 4, **characterised in that** the lens is attached to the light-converting element (18, 19).

6. Apparatus according to any one of claims 3 to 5, **characterised in that** the lenses of the two transmitting units (11, 12) are oriented parallel to each other, wherein the centre axes of the lenses are oriented transversely to the direction of conveying F of the products (15).

7. Apparatus according to any one of claims 1 to 6, **characterised in that** the distance between the two lines of light generated by the light sources (16, 17) or lenses is approximately 40 mm.

8. Apparatus according to any one of claims 1 to 7, **characterised in that** the detection means (22) comprises a camera, wherein the detection means (22) is assigned an evaluating unit.

9. Apparatus according to any one of claims 1 to 8, **characterised in that** the light-converting element (18, 19) is constructed and designed to form a rectangular light field.

10. Apparatus according to any one of claims 1 to 9, **characterised in that** both transmitting units (11, 12) are arranged above the transport element (14) for conveying the products (15).

11. Method for the contactless detection of characteristics of continuously conveyed, translucent products (15) with an apparatus according to one or more of claims 1 to 10, comprising the steps of:
- continuously conveying the products (15) through an inspection region of a receiving unit (13),
- exposing the products (15) to light radiation by means of a transmitting unit (11, 12), and
- picking up the light radiation transflected by the products (15) by means of the receiving unit (13),
**characterised in that** the products (15) are exposed to high-intensity light radiation from separate light sources (16, 17) both on entering the inspection region of a detection means (22) of the receiving unit (13) and on leaving the inspection region of the detection means (22).

12. Method according to claim 11, **characterised in that** the light beams emanating from the light sources (16, 17) are first widened into a planar light field by a light-converting element (18, 19), and then focused by a focusing element (20, 21) into a line of light running transversely to the direction of conveying F of the products (15), wherein the light beams are shielded on at least two sides.

13. Method according to claim 11 or 12, **characterised in that** the transflected light radiation picked up by means of the receiving unit (13) is processed by means of an evaluating unit.

## Revendications

1. Dispositif (10) pour la détection sans contact de caractéristiques de produits translucides (15) transportés en continu, comprenant d'une part une unité d'émission (11, 12) avec une source de lumière (16, 17) pour générer un rayonnement de lumière à haute intensité, un élément de transformation de lumière (18, 19) pour la formation d'un champ de lumière surfacique à partir du rayonnement de lumière et un élément de focalisation (20, 21) pour former une ligne de lumière s'étendant transversalement à la direction de transport F des produits (15) à partir du champ de lumière surfacique, et d'autre part une unité de réception (13) avec un moyen de détection (22) pour recevoir le rayonnement de lumière transfléchi par le produit (15), dans lequel un élément d'obscurcissement (23, 24) est agencé entre l'unité d'émission (11, 12) et l'unité de réception (13), **caractérisé en ce qu'**au moins deux unités d'émission (11, 12) sont prévues avec une structure correspondante, **en ce qu'**au moins deux sources de lumière indépendantes (16, 17) sont prévues pour éclairer le produit (15), dans lequel une unité d'émission (12) est agencée avant l'unité de réception (13) dans la direction de transport F des produits (15) et l'autre unité d'émission (11) est agencée après l'unité de réception (13) dans la direction de transport F, dans lequel les deux unités d'émission (11, 12) sont protégées de l'unité de réception (13) au moyen de l'élément d'obscurcissement (23, 24), dans lequel l'unité de réception (13) est intercalée entre les éléments d'obscurcissement (23, 24) orientés verticalement par rapport au plan de transport des produits (15).

2. Dispositif selon la revendication 1, **caractérisé en ce que** au moins deux éléments d'obscurcissement (23, 25 ; 24, 26) sont associés à chaque unité d'émission (11, 12) de telle sorte que les unités d'émission (11, 12) sont protégées chacune des deux côtés au moyen d'un élément d'obscurcissement (23, 25 ; 24, 26).

3. Dispositif selon la revendication 1 ou 2, **caractérisé en ce que** l'élément de focalisation (20, 21) est une lentille cylindrique.

4. Dispositif selon la revendication 3, **caractérisé en ce que** la lentille est constituée de polyméthacrylates et a un diamètre de 25 mm et une longueur de 200 mm.

5. Dispositif selon la revendication 3 ou 4, **caractérisé en ce que** la lentille est fixée à l'élément de transformation de lumière (18, 19).

6. Dispositif selon l'une quelconque des revendications 3 à 5, **caractérisé en ce que** les lentilles des deux unités d'émission (11, 12) sont orientées parallèlement, dans lequel les axes centraux des lentilles sont orientés transversalement à la direction de transport F des produits (15).

7. Dispositif selon l'une quelconque des revendications 1 à 6, **caractérisé en ce que** la distance entre les deux lignes de lumière générées par les sources de lumière (16, 17) ou les lentilles est d'environ 40 mm.

8. Dispositif selon l'une quelconque des revendications 1 à 7, **caractérisé en ce que** le moyen de détection (22) comprend une caméra, auquel moyen de détection (22) est associée une unité d'évaluation.

9. Dispositif selon l'une quelconque des revendications 1 à 8, **caractérisé en ce que** l'élément de transformation de lumière (18, 19) est conçu et configuré pour former un champ de lumière rectangulaire.

10. Dispositif selon l'une quelconque des revendications 1 à 9, **caractérisé en ce que** les deux unités d'émission (11, 12) sont agencées au-dessus d'un élément de transport (14) destiné à transporter les produits (15).

11. Procédé pour la détection sans contact de caractéristiques de produits translucides (15) transportés en continu, à l'aide d'un dispositif selon une ou plusieurs des revendications 1 à 10, comprenant les étapes suivantes :
- transporter en continu les produits (15) à travers une zone d'inspection d'une unité de réception (13),
- irradier les produits (15) avec un rayonnement de lumière au moyen d'une unité d'émission (11, 12), et
- recevoir le rayonnement de lumière transfléchi par les produits (15) au moyen de l'unité de réception (13),
**caractérisé en ce que** les produits (15) sont irradiés par un rayonnement de lumière à haute intensité provenant de deux sources de lumière séparées (16, 17) à la fois à l'entrée dans la zone d'inspection d'un moyen de détection (22) de l'unité de réception (13) et à la sortie de la zone d'inspection du moyen de détection (22).

12. Procédé selon la revendication 11, **caractérisé en ce que** les faisceaux de lumière issus des sources de lumière (16, 17) sont d'abord élargis par un élément de transformation de lumière (18, 19) pour former un champ de lumière surfacique, et ensuite focalisés par un élément de focalisation (20, 21) en une ligne de lumière s'étendant transversalement à la direction de transport F des produits (15), dans lequel les faisceaux de lumière font l'objet d'une protection sur au moins deux côtés.

13. Procédé selon la revendication 11 ou 12, **caractérisé en ce qu'**un rayonnement de lumière transfléchi enregistré au moyen de l'unité de réception (13) est traité au moyen d'une unité d'évaluation.
